# EUROPEAN PATENT APPLICATION

(11) **EP 4 302 715 A1**
(43) Date of publication of application: **10.01.2024**
(21) Application number: 22767032.0
(22) Date of filing: 04.03.2022
(51) Int. Cl.: A61B 18/14

(54) **MEDICAL DEVICE**

(30) Priority: 08.03.2021 JP 2021036373
(71) Applicant: TERUMO Kabushiki Kaisha, Tokyo 151-0072 (JP)
(72) Inventor: FUKAMI, Kazunari, Ashigarakami-gun, Kanagawa 259-0151 (JP); TAKAHASHI, Yusuke, Ashigarakami-gun, Kanagawa 259-0151 (JP)
(74) Representative: Casalonga
(86) International application number: PCT/JP2022/009468
(87) International publication number: WO 2022/191075

(57) **Abstract**

Provided is a medical device which, when a biological tissue is clamped by an expansion body, can adjust the pressing force applied on the biological tissue by an electrode portion to be appropriate. The present invention comprises an expansion body 21, a shaft portion 20, and an electrode portion 22. The expansion body 21 has a recess 51. The recess 51 comprises a bottom portion 51a, a proximal-side upright portion 52, and a distal-side upright portion 53. One of the proximal-side upright portion 52 and the distal-side upright portion 53 is provided with the electrode portion 22, while the other of the proximal-side upright portion 52 and the distal-side upright portion 53 is provided with: a bifurcated outer peripheral portion 55; a back support portion 56 which has a receiving surface 56a and which is disposed inside the outer peripheral portion 55; and an arm portion 57 which extends from the back support portion 56. The arm portion 57 is connected to the outer peripheral portion 55, and is deformed to limit an inclination amount of the receiving surface 56a of the back support portion 56 when the receiving surface 56a is inclined.

## Description

### Technical Field

The present invention relates to a medical device that applies energy to a biological tissue.

### Background Art

A medical device is known that includes an electrode portion provided on an expansion body that expands and contracts in a living body, and performs treatment by ablation for cauterizing a biological tissue by a high-frequency current from the electrode portion. As one of treatments by ablation, a shunt treatment on the atrial septum is known. The shunt treatment can alleviate heart failure symptoms of a patient with heart failure by forming a shunt (puncture hole) serving as an escape route for increased atrial pressure in the atrial septum of the patient. In the shunt treatment, the atrial septum is accessed using an intravenous approaching method, and a puncture hole with a desired size is formed. Such a medical device is disclosed in, for example, Patent Literature 1.

### Citation List

### Patent Literature

Patent Literature 1: WO 2020/94087 A

### Summary of Invention

### Technical Problem

In the medical device, the expansion body has: a recess recessed radially inward during expansion and defining a reception space configured to receive a biological tissue; and an electrode portion provided on a proximal-side upright portion or a distal-side upright portion that defines the recess. When the biological tissue is clamped in the recess, the electrode portion moves to be pressed against the biological tissue, and a receiving surface which is a surface of the recess on a side opposite to a side where the electrode portion is provided also moves in the same direction as the electrode portion by the pressing force. When the expansion body is formed of a mesh as in Patent Literature 1, both the electrode portion and the receiving surface can move in the same direction. However, due to the mesh expansion body being likely to be deformed, the shape of the recess is distorted to make it impossible to sufficiently cauterize, and thus, it may not be possible to form a shunt with a desired size. On the other hand, when the expansion body is rigid, the electrode portion and the receiving surface may be twisted, which may decrease the pressing force of the electrode portion against the biological tissue.

The present invention has been made to solve the above-described problems, and an object of the present invention is to provide a medical device that can adjust a pressing force of an electrode portion against a biological tissue to be appropriate when the biological tissue is clamped by an expansion body.

### Solution to Problem

In order to achieve the above object, a medical device according to the present invention includes: an expansion body that is expandable and contractible in a radial direction; a shaft portion that is elongated and includes a distal end part including a proximal end fixing portion to which a proximal end of the expansion body is fixed; and an electrode portion provided along the expansion body, in which the expansion body includes a recess that is recessed radially inward during expansion of the expansion body and defines a reception space configured to receive a biological tissue, the recess includes a bottom portion located on an innermost side in the radial direction, a proximal-side upright portion extending outward in the radial direction from a proximal end of the bottom portion, and a distal-side upright portion extending outward in the radial direction from a distal end of the bottom portion, the electrode portion is provided on one of the proximal-side upright portion and the distal-side upright portion, another of the proximal-side upright portion and the distal-side upright portion is provided with an outer peripheral portion that is bifurcated from the vicinity of the bottom portion and extends outward in the radial direction, a back support portion that is disposed inside the outer peripheral portion and includes a receiving surface facing the electrode portion when the expansion body expands, and an arm portion extending from the back support portion or from the outer peripheral portion, the receiving surface of the back support portion is inclined so as to be substantially parallel to the electrode portion via an object clamped between the electrode portion and the back support portion when the electrode portion moves toward the back support portion, and the arm portion includes any one of configurations of i) extending from the back support portion, while curving or bending, to be connected to the outer peripheral portion, and being deformed to limit an inclination amount of the receiving surface of the back support portion when the receiving surface is inclined, ii) extending from the back support portion, and coming into contact with the outer peripheral portion or a part of the expansion body to limit an inclination amount of the receiving surface of the back support portion when the receiving surface is inclined, iii) extending from the outer peripheral portion, and coming into contact with the back support portion to limit an inclination amount of the back support portion when the receiving surface of the back support portion is inclined, and iv) connecting the back support portion and the outer peripheral portion so as to form a U-shape open toward the bottom portion together with the back support portion, and rotating about a connection portion with the outer peripheral portion to limit an inclination amount of the receiving surface of the back support portion when the receiving surface is inclined.

### Advantageous Effects of Invention

In the medical device configured as described above, the arm portion limits the inclination amount when the receiving surface of the back support portion is inclined. Thus, the back support portion is not excessively inclined, and the pressing force of the electrode portion against the biological tissue when the biological tissue is clamped between the electrode portion and the receiving surface can be adjusted to be appropriate. This makes it possible to cauterize the biological tissue against which the electrode portion is sufficiently pressed, whereby the impedance during conduction of electricity is reliably increased, and it is possible to clearly determine the end of cauterization. In addition, the biological tissue can be effectively cauterized, whereby a shunt having a stable size can be formed. In addition, the electrode portion is in close contact with the biological tissue and the back support portion, whereby it is possible to prevent transmission of energy from the electrode portion to blood or other biological tissues.

The back support portion may extend outward in the radial direction in a plate shape from the outer peripheral portion near the bottom portion. With this configuration, the receiving surface of the back support portion can be more reliably inclined substantially parallel to the electrode portion.

The arm portion may be bifurcated from a radially outer end of the back support portion, and extend symmetrically about an extending direction of the back support portion, while curving or bending, to be connected to the outer peripheral portion, the arm portion being deformed to limit an inclination amount of the receiving surface of the back support portion when the receiving surface is inclined. This configuration can prevent the receiving surface of the back support portion from being twisted to the left or right when the receiving surface is inclined, whereby the biological tissue can be reliably clamped between the electrode portion and the back support portion.

The arm portion may extend from a radially outer end of the back support portion, and come into contact with the outer peripheral portion to limit an inclination amount of the receiving surface of the back support portion when the receiving surface is inclined. With this configuration, the arm portion comes in contact with the outer peripheral portion, whereby the inclination amount of the receiving surface is reliably limited.

The outer peripheral portion may include a merging portion at which merging occurs at an end opposite to the bottom portion, the expansion body may include an extension portion extending radially inward from the merging portion of the outer peripheral portion, and the arm portion may extend from a radially outer end of the back support portion, and come into contact with an inner surface of the extension portion to limit an inclination amount of the receiving surface of the back support portion when the receiving surface is inclined. With this configuration, the arm portion comes into contact with the inner surface of the extension portion, whereby the inclination amount of the receiving surface is reliably limited.

The arm portion may extend to connect bifurcated parts of the outer peripheral portion, and come into contact with a surface of the back support portion opposite to a surface facing the electrode portion to limit an inclination amount of the receiving surface of the back support portion when the receiving surface is inclined. With this configuration, the inclination amount of the receiving surface is reliably limited by the arm portion provided on the outer peripheral portion.

The outer peripheral portion may include a merging portion at which merging occurs at an end opposite to the bottom portion, and the arm portion may extend toward the back support portion from the merging portion of the outer peripheral portion, and come into contact with a surface of the back support portion opposite to a surface facing the electrode portion to limit an inclination amount of the receiving surface of the back support portion when the receiving surface is inclined. With this configuration, the inclination amount of the receiving surface is reliably limited by the arm portion extending from the merging portion.

The back support portion may include a plurality of rods extending substantially perpendicular to a radial direction of the expansion body and substantially parallel to a surface of the electrode portion facing the recess, the plurality of rods being disposed substantially parallel to each other, the arm portion may include a plurality of connecting arm portions that connect both ends of the plurality of rods and the outer peripheral portion so as to form a U-shape open toward the bottom portion together with the plurality of rods, the receiving surface of the back support portion may be defined by a virtual plane including at least a part of each of the plurality of rods, and each of the plurality of connecting arm portions may rotate about a connection portion with the outer peripheral portion to limit an inclination amount of the receiving surface of the back support portion when the receiving surface is inclined. With this configuration, the inclination amount of the receiving surface of the back support portion including the plurality of rods is reliably limited by the connecting arm portions.

### Brief Description of Drawings

Fig. 1 is a front view illustrating an overall configuration of a medical device according to an embodiment.
Fig. 2 is an enlarged perspective view illustrating the vicinity of an expansion body.
Fig. 3 is a developed view of the vicinity of a recess of the expansion body.
Fig. 4 is an enlarged perspective view illustrating the vicinity of a back support portion of the expansion body.
Fig. 5 is an explanatory diagram for schematically describing a state in which the expansion body is placed in the atrial septum, Fig. 5 including a front view of the medical device and a cross-sectional view of a biological tissue.
Fig. 6 is a flowchart of a treatment using the medical device.
Fig. 7 is a diagram illustrating a state in S2 in Fig. 6, in which Fig. 7(a) is an enlarged view of the vicinity of a balloon in atrial septum illustrated in cross section, and Fig. 7(b) is a cross-sectional view of the atrial septum for describing a shape of a puncture hole.
Fig. 8 is a diagram illustrating a state in S3 in Fig. 6, in which Fig. 8(a) is an enlarged view of the vicinity of the expansion body illustrating the atrial septum in cross section and the inside of a storage sheath in a transparent manner, and Fig. 8(b) is a cross-sectional view of the atrial septum in a state where the storage sheath is inserted through a puncture hole.
Fig. 9 is a diagram illustrating a state in S4 in Fig. 6, and is an enlarged view of the vicinity of the expansion body in the atrial septum illustrated in cross section.
Fig. 10 is a diagram illustrating a state in S4 in Fig. 6, and is a cross-sectional view of the atrial septum in a state where the puncture hole is enlarged with the expansion body.
Fig. 11 is a diagram illustrating a state in S5 in Fig. 6, and is an enlarged view of the vicinity of the expansion body in the atrial septum illustrated in cross section.
Fig. 12 is an enlarged cross-sectional view illustrating the atrial septum and the expansion body, in which Fig. 12(a) illustrates a state before the atrial septum is clamped between a distal-side upright portion and a proximal-side upright portion, and Fig. 12(b) illustrates a state in which the atrial septum is clamped between the distal-side upright portion and the proximal-side upright portion.
Fig. 13 is a diagram illustrating the atrial septum and the expansion body, in which Fig. 13(a) is a cross-sectional view taken along line A-A in Fig. 12(a), and Fig. 13(b) is a cross-sectional view taken along line B-B in Fig. 12(b).
Fig. 14 is an enlarged perspective view of the vicinity of a back support portion of an expansion body according to a first modification.
Fig. 15 is an enlarged perspective view of the vicinity of a back support portion of an expansion body according to a second modification.
Fig. 16 is an enlarged perspective view of the vicinity of a back support portion of an expansion body according to a third modification.
Fig. 17 is an enlarged perspective view of the vicinity of a back support portion of an expansion body according to a fourth modification.
Fig. 18 is an enlarged perspective view of the vicinity of a back support portion of an expansion body according to a fifth modification.
Fig. 19 is an enlarged perspective view of the vicinity of a back support portion of an expansion body according to a sixth modification.
Fig. 20 is an enlarged perspective view of the vicinity of a back support portion of an expansion body according to a seventh modification.
Fig. 21 is an enlarged perspective view of the vicinity of a back support portion of an expansion body according to an eighth modification.
Fig. 22 is an enlarged perspective view of the vicinity of a back support portion of an expansion body according to a ninth modification.
Fig. 23 is a cross-sectional view illustrating a state in which the atrial septum is clamped between a proximal-side upright portion and a distal-side upright portion of the expansion body according to the ninth modification.

### Description of Embodiments

An embodiment of the present invention will be described below with reference to the drawings. Note that dimensional ratios in the drawings may be exaggerated and different from actual ratios for convenience of description. In addition, in the present specification, a side on which a medical device 10 is inserted into a biological lumen will be referred to as "distal end" or "distal side", and a side operated by an operator will be referred to as "proximal end" or "proximal side".

The medical device according to the embodiment described below is configured to expand a puncture hole Hh formed in an atrial septum HA of the heart H of a patient, and to further perform a maintenance treatment to keep the expanded puncture hole Hh at the increased size.

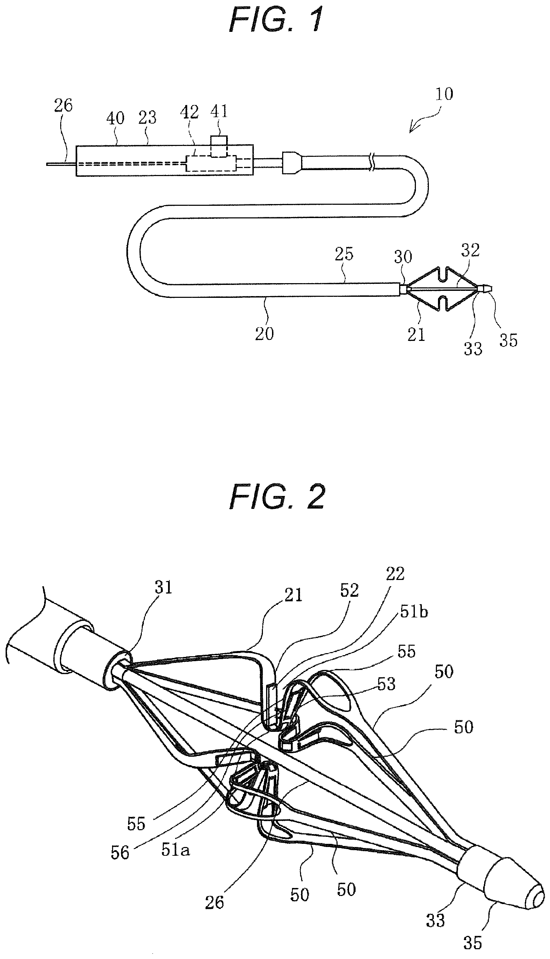

As illustrated in Fig. 1, the medical device 10 according to the present embodiment includes an elongated shaft portion 20, an expansion body 21 disposed at a distal end part of the shaft portion 20, and a hand operation unit 23 disposed at a proximal end part of the shaft portion 20. The expansion body 21 has an electrode portion 22 which is an energy transfer element for performing the above-described maintenance treatment.

The shaft portion 20 has a distal end part 30 including a proximal end fixing portion 31 to which a proximal end of the expansion body 21 is fixed and a distal end fixing portion 33 to which a distal end of the expansion body 21 is fixed. The distal end part 30 of the shaft portion 20 has a shaft extension portion 32 extending within the expansion body 21 from the proximal end fixing portion 31. The shaft portion 20 has a storage sheath 25 disposed at an outermost peripheral portion. The expansion body 21 is movable forward and rearward in an axial direction relative to the storage sheath 25. The storage sheath 25 can accommodate the expansion body 21 therein in a state of moving to the distal side of the shaft portion 20. The expansion body 21 can be exposed by moving the storage sheath 25 that has accommodated the expansion body 21 to the proximal side.

The shaft portion 20 includes a pulling shaft 26. The pulling shaft 26 is disposed from the proximal end of the shaft portion 20 to the shaft extension portion 32, and the distal end part thereof is fixed to a distal member 35.

The distal member 35 to which the distal end part of the pulling shaft 26 is fixed may not be fixed to the expansion body 21. Accordingly, the distal member 35 can pull the expansion body 21 in a contracting direction. In addition, when the expansion body 21 is stored in the storage sheath 25, the distal member 35 is separated to the distal side from the expansion body 21, by which the expansion body 21 can be rather easily moved in an axial direction. Thus, ease of storage can be improved.

The hand operation unit 23 has a housing 40 gripped by an operator, an operation dial 41 that can be rotated by the operator, and a conversion mechanism 42 that operates in conjunction with the rotation of the operation dial 41. The pulling shaft 26 is held by the conversion mechanism 42 inside the hand operation unit 23. The conversion mechanism 42 can move the pulling shaft 26 held by the conversion mechanism 42 forward and backward along the axial direction in conjunction with the rotation of the operation dial 41. A rack and pinion mechanism can be used as the conversion mechanism 42, for example.

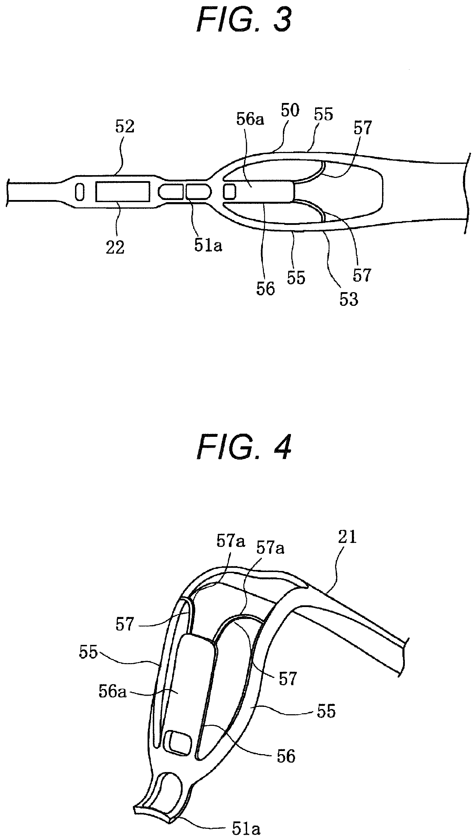

The expansion body 21 will be described in more detail. As illustrated in Figs. 2 and 3, the expansion body 21 has a plurality of wire portions 50 in a circumferential direction. In the present embodiment, four wire portions 50 are disposed in the circumferential direction. Each of the wire portions 50 is configured to expand and contract in a radial direction. A proximal end part of the wire portion 50 extends to the distal side from the proximal end fixing portion 31. A distal end part of the wire portion 50 extends to the proximal side from a proximal end part of the distal end fixing portion 33. The wire portion 50 is inclined to increase in the radial direction from both ends toward a central part in the axial direction. In addition, the wire portion 50 has a recess 51 in the central part in the axial direction, the recess 51 being recessed radially inward of the expansion body 21. An innermost part of the recess 51 in the radial direction is a bottom portion 51a. The recess 51 defines a reception space 51b configured to receive a biological tissue when the expansion body 21 expands.

The recess 51 includes a proximal-side upright portion 52 extending radially outward from the proximal end of the bottom portion 51a and a distal-side upright portion 53 extending radially outward from the distal end of the bottom portion 51a. The electrode portion 22 is disposed on the proximal-side upright portion 52 or the distal-side upright portion 53 so as to face the reception space 51b. The distal-side upright portion 53 includes outer peripheral portions 55 bifurcated from the vicinity of the bottom portion 51a and extending radially outward, and a back support portion 56 disposed between the two outer peripheral portions 55. The back support portion 56 has a receiving surface 56a facing the electrode portion 22 when the expansion body 21 expands. The back support portion 56 has a plate shape.

The expansion body 21 has arm portions 57 that extend from the back support portion 56 while curving and are connected to the outer peripheral portions 55 as illustrated in Figs. 3 and 4. The arm portions 57 are bifurcated from the radially outer end of the back support portion 56, and extend symmetrically about the extending direction of the back support portion 56 while being curved. Therefore, each of the arm portions 57 has a curved bent portion 57a. Note that the bent portion 57a of the arm portion 57 may be cranked.

The wire portion 50 forming the expansion body 21 has, for example, a flat plate shape cut from a cylinder. The wire forming the expansion body 21 can have a thickness in a range of 50 µm to 500 µm and a width in a range of 0.3 mm to 2.0 mm. However, the wire may have a dimension outside this range. In addition, the wire portion 50 may have a circular shape in a cross section, or may have other shapes in a cross section.

The electrode portion 22 includes, for example, a bipolar electrode that receives electric energy from an energy supply device (not illustrated) serving as an external device. In this case, electricity is conducted between the electrode portions 22 disposed on the wire portions 50. The electrode portion 22 and the energy supply device are connected to each other by a conductive wire (not illustrated) coated with an insulating coating material. The conductive wire is drawn out to the outside via the shaft portion 20 and the hand operation unit 23, and is connected to the energy supply device.

Alternatively, the electrode portion 22 may be configured as a monopolar electrode. In this case, electricity is supplied from a counter electrode plate prepared outside a body. In addition, a heating element (electrode chip) that generates heat by receiving high-frequency electric energy from the energy supply device may be used instead of the electrode portion 22. In this case, electricity is conducted between the heating elements disposed on the wire portions 50. Furthermore, the electrode portion 22 can be constituted by an energy transfer element configured to apply energy to the puncture hole Hh, such as a heater including an electric wire which provides heating and cooling operation or generating frictional heat by using microwave energy, ultrasound energy, coherent light such as laser, a heated fluid, a cooled fluid, or a chemical medium. A specific form of the energy transfer element is not particularly limited.

The wire portion 50 can be formed of a metal material. Examples of the metal material which can be used include a titanium-based (Ti-Ni, Ti-Pd, or Ti-Nb-Sn) alloy, a copper-based alloy, stainless steel, β-titanium steel, and a Co-Cr alloy. Note that an alloy having a spring property such as a nickel titanium alloy may be more preferably used. However, a material of the wire portion 50 is not limited to the above materials, and the wire portion 50 may be formed of other materials.

It is preferable that the shaft portion 20 is formed of a material having a certain degree of flexibility. Examples of such a material include polyolefin such as polyethylene, polypropylene, polybutene, ethylene-propylene copolymer, ethylene-vinyl acetate copolymer, ionomer, or a mixture of two or more of them, soft polyvinyl chloride resin, polyamide, polyamide elastomer, polyester, polyester elastomer, polyurethane, fluorine resin such as polytetrafluoroethylene, polyimide, PEEK, silicone rubber, and latex rubber.

The pulling shaft 26 can be formed of, for example, an elongated wire material including a super elasticity alloy such as a nickel-titanium alloy and a copper-zinc alloy, a metal material such as stainless steel, and a resin material having comparatively high rigidity.

The distal member 35 can be formed of, for example, a super elasticity alloy such as a nickel-titanium alloy or a copper-zinc alloy, a metal material such as stainless steel, a polymer material such as polyolefin, polyvinyl chloride, polyamide, polyamide elastomer, polyurethane, polyurethane elastomer, polyimide, and fluororesin or a mixture of the above polymer materials. Alternatively, the distal member 35 can be formed of a multilayer tube containing two or more kinds of polymer materials.

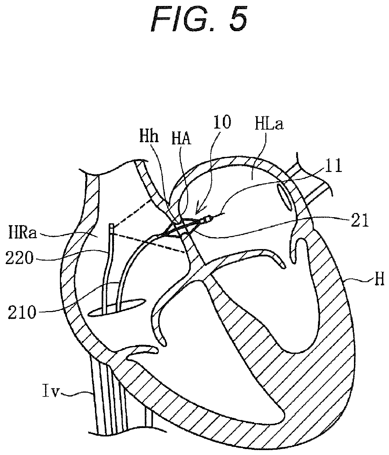

A treatment method using the medical device 10 will be described. The treatment method according to the present embodiment is performed on a patient suffering from a heart failure (left heart failure). More specifically, the treatment method is performed on the patient with a chronic heart failure having a high blood pressure in a left atrium HLa due to myocardial hypertrophy appearing in a left ventricle of the heart H and increased stiffness (hardness) as illustrated in Fig. 5.

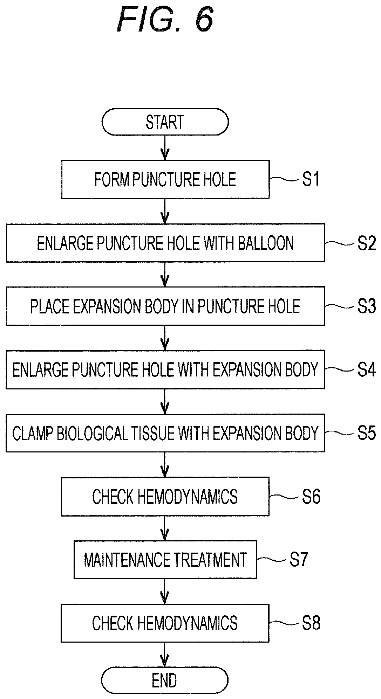

First, a puncture hole Hh is formed in the atrial septum HA (S1) as illustrated in Fig. 6. In order to form the puncture hole Hh, an operator delivers an introducer 210 obtained by combining a guiding sheath and a dilator to the vicinity of the atrial septum HA. The introducer 210 can be delivered to a right atrium HRa via, for example, an inferior vena cava Iv. In addition, the introducer can be delivered using the guide wire 11. The operator can insert the guide wire 11 into the dilator and deliver the introducer along the guide wire 11. Note that the introducer and the guide wire 11 can be inserted into a living body with a known method such as a method for using an introducer to be introduced into a blood vessel.

The operator inserts a puncture device (not illustrated) so that the puncture device penetrates from the right atrium HRa side toward the left atrium HLa side, thereby forming the puncture hole Hh. The puncture device is inserted into the dilator and delivered to the atrial septum HA.

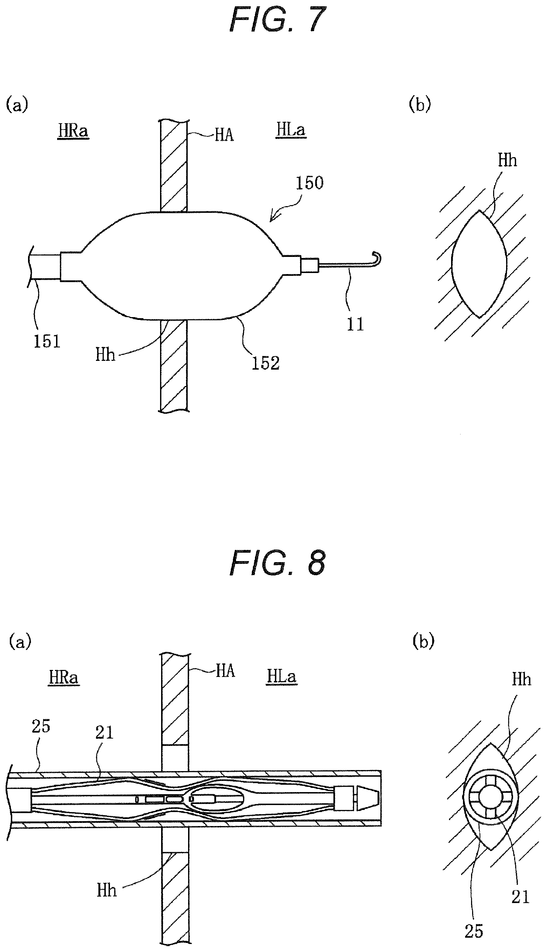

Next, the operator delivers a balloon catheter 150 to the vicinity of the atrial septum HA along the guide wire 11 inserted in advance. The balloon catheter 150 includes a balloon 152 at a distal end part of a shaft portion 151 as illustrated in Fig. 7. After placing the balloon 152 in the atrial septum HA, the operator expands the balloon in the radial direction to enlarge the puncture hole Hh (S2) as illustrated in Fig. 7(a). During this process, the puncture hole Hh is enlarged to a size equal to the maximum diameter of the expanded balloon 152 in the direction along fibers of the septal tissue due to the influence of the fibers, but is less likely to enlarge in other directions. Therefore, the puncture hole Hh has an elongated shape as illustrated in Fig. 7(b).

Next, the operator delivers the medical device 10 to the vicinity of the atrial septum HA and place the expansion body 21 at the position of the puncture hole Hh (S3). Although the guide wire is not used for the delivery of the medical device 10, the guide wire may be used for stable operation with the heart beating. At this time, the distal end part of the medical device 10 penetrates the atrial septum HA and reaches the left atrium HLa. In addition, when the medical device 10 is inserted, the expansion body 21 is in a state of being stored in the storage sheath 25 as illustrated in Fig. 8(a). Since the puncture hole Hh is enlarged by the balloon 152, the storage sheath 25 can be inserted into the puncture hole Hh as illustrated in Fig. 8(b).

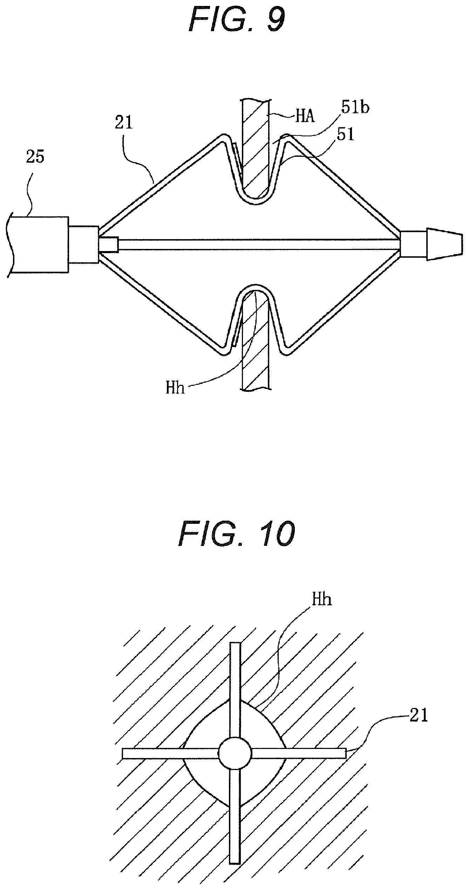

Next, the operator moves the storage sheath 25 to the proximal side, thereby exposing the expansion body 21 as illustrated in Fig. 9. In this manner, the expansion body 21 increases in diameter, and the recess 51 is located in the puncture hole Hh of the atrial septum HA and receives the biological tissue surrounding the puncture hole Hh in the reception space 51b (S4). Due to the expansion of the expansion body 21, the puncture hole Hh is enlarged to have a substantially uniform diameter along the circumferential direction as illustrated in Fig. 10. The expansion body 21 changes the shape of the puncture hole Hh, but does not increase the maximum diameter. Therefore, the maximum diameter of the puncture hole Hh is equal to the diameter of the puncture hole Hh enlarged by the balloon 152 in S2 in the major axis direction.

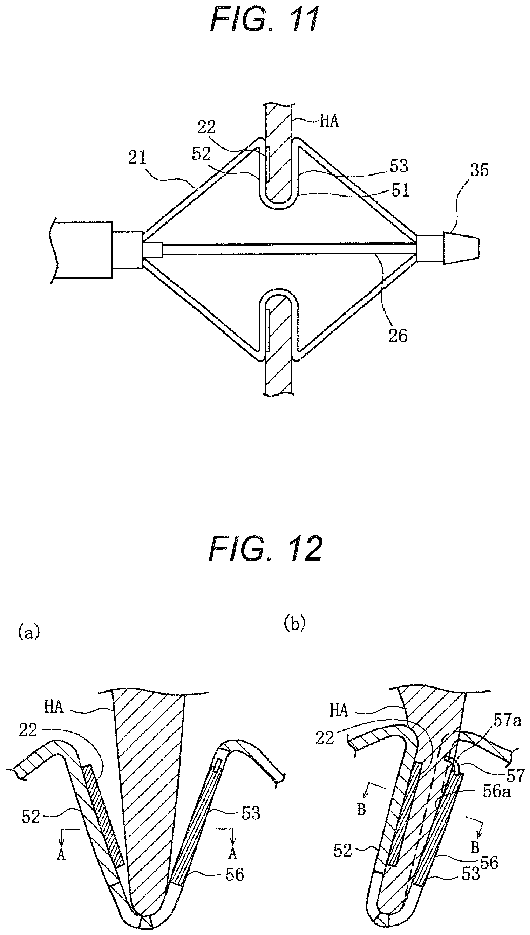

The operator operates the operation unit 23 with the biological tissue being received in the reception space 51b, and moves the pulling shaft 26 to the proximal side. With this operation, the expansion body 21 is contracted in the axial direction by being pulled in the contracting direction by the distal member 35, so that the atrial septum HA is held by the proximal-side upright portion 52 and the distal-side upright portion 53, and the electrode portion 22 is pressed against the biological tissue (S5) as illustrated in Fig. 11.

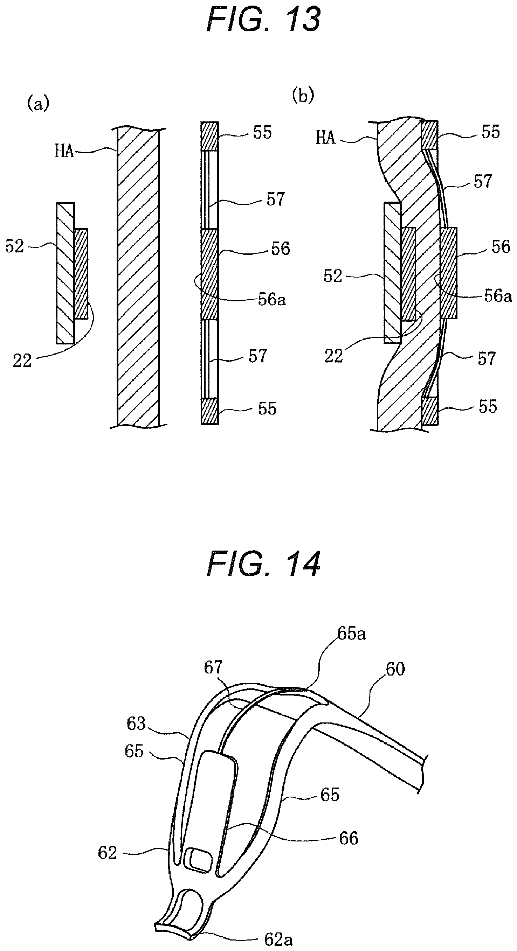

When the proximal-side upright portion 52 and the distal-side upright portion 53 which have been separated from each other as illustrated in Figs. 12(a) and 13(a) are brought close to each other, the atrial septum HA is clamped between the proximal-side upright portion 52 and the distal-side upright portion 53 as illustrated in Figs. 12(b) and 13(b). Then, the electrode portion 22 presses the atrial septum HA toward the distal side. At this time, the distal-side upright portion 53 inclines the back support portion 56 toward the distal side between the two outer peripheral portions 55, thereby receiving the atrial septum HA pressed by the electrode portion 22 between the two outer peripheral portions 55. The receiving surface 56a of the back support portion 56 receives a force from the electrode portion 22 via the atrial septum HA, and is inclined so as to be substantially parallel to the electrode portion 22. Then, the back support portion 56 applies, to the atrial septum HA pushed by the electrode portion 22, a repulsive force in the direction opposite to the direction in which the electrode portion 22 pushes the atrial septum HA, while flexibly bending. As a result, the electrode portion 22 is in close contact with the atrial septum HA. At this time, the arm portions 57 are deformed with the inclination of the back support portion 56. The arm portions 57 each has the bent portion 57a, and thus, the arm portions are deformed by a certain amount in the pushing direction of the electrode portion 22 and are not deformed any more. When the arm portions 57 are not deformed any more, the back support portion 56 cannot be further inclined. That is, the amount of inclination of the back support portion 56 is limited by the arm portions 57. Accordingly, the pushing force of the electrode portion 22 can be sufficiently transmitted to the atrial septum HA.

After enlarging the puncture hole Hh, the operator checks hemodynamics (S6). The operator delivers a hemodynamics checking device 220 to the right atrium HRa by way of the inferior vena cava Iv as illustrated in Fig. 5. As the hemodynamics checking device 220, a known echo catheter can be used, for example. The operator can display an echo image acquired by the hemodynamics checking device 220 on a display device such as a display, and can check an amount of blood passing through the puncture hole Hh on the basis of the displayed echo image.

Next, the operator performs the maintenance treatment for maintaining the size of the puncture hole Hh (S7). During the maintenance treatment, high-frequency energy is applied to an edge of the puncture hole Hh through the electrode portion 22 to cauterize (heat and cauterize) the edge of the puncture hole Hh by the high-frequency energy. The high-frequency energy is imparted by applying a voltage across the electrode portions 22 adjacent to each other in the circumferential direction. At this time, the back support portion 56 is inclined against the pushing force of the electrode portion 22 with the amount of inclination being limited by the arm portions 57 as described above, so that the cauterization is performed in a state where the electrode portion 22 is sufficiently pressed against the atrial septum HA. Therefore, the impedance is reliably increased, and the end of cauterization can be clearly determined. In addition, the atrial septum HA can be effectively cauterized, and a shunt having a stable size can be formed. In addition, since the electrode portion 22 is in close contact with the atrial septum HA and the back support portion 56, it is possible to prevent transmission of energy from the electrode portion 22 to the blood or other biological tissues.

When the biological tissue in the vicinity of the edge of the puncture hole Hh is cauterized through the electrode portion 22, a degenerated portion where the biological tissue is degenerated is formed in the vicinity of the edge. The biological tissue in the degenerated portion loses elasticity, and thus, the puncture hole Hh can maintain the shape enlarged by the expansion body 21.

After performing the maintenance treatment, the operator checks the hemodynamics again (S8). When the amount of blood passing through the puncture hole Hh reaches a desired amount, the operator contracts the expansion body 21, stores the expansion body 21 into the storage sheath 25, and then, removes the expansion body 21 from the puncture hole Hh. Furthermore, the operator removes the entire medical device 10 from the living body to the outside, and ends the treatment.

Next, an expansion body according to modifications will be described. As illustrated in Fig. 14, a distal-side upright portion 63 of an expansion body 60 according to a first modification is provided with outer peripheral portions 65 and a back support portion 66. The outer peripheral portions 65 have a merging portion 65a where the outer peripheral portions 65 merge at an end of a recess 62 opposite to a bottom portion 62a. The expansion body 60 has an arm portion 67 that extends from the back support portion 66 while curving and is connected to the merging portion 65a of the outer peripheral portions 65. A single arm portion 67 extending from the radially outer end of the back support portion 56 to the merging portion 65a is provided. Due to the arm portion 67 having the above configuration, the inclination amount of the back support portion 66 can also be limited.

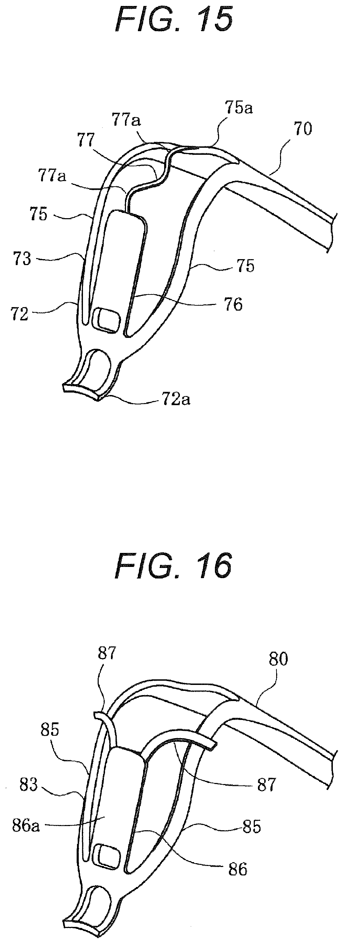

As illustrated in Fig. 15, a distal-side upright portion 73 of an expansion body 70 according to a second modification is provided with outer peripheral portions 75 and a back support portion 76. The outer peripheral portions 75 have a merging portion 75a where the outer peripheral portions 75 merge at an end of a recess 72 opposite to a bottom portion 72a. The expansion body 70 has an arm portion 77 that extends from the back support portion 76 while curving and is connected to the merging portion 75a of the outer peripheral portions 75. The arm portion 77 has a plurality of bent portions 77a. As described above, the plurality of bent portions 77a of the arm portion 77 may be provided.

As illustrated in Fig. 16, a distal-side upright portion 83 of an expansion body 80 according to a third modification is provided with outer peripheral portions 85 and a back support portion 86. The expansion body 80 has arm portions 87 extending from the back support portion 86. The arm portions 87 extend in two directions from the radially outer end of the back support portion 86 so as to be symmetrical about the extending direction of the back support portion 86 while curving. The arm portions 87 are spaced apart from outer peripheral portions 85 and other portions of the expansion body 80. When a receiving surface 86a of the back support portion 86 is inclined, the arm portions 87 come in contact with the outer peripheral portions 85, so that the inclination amount of the back support portion 86 can be limited.

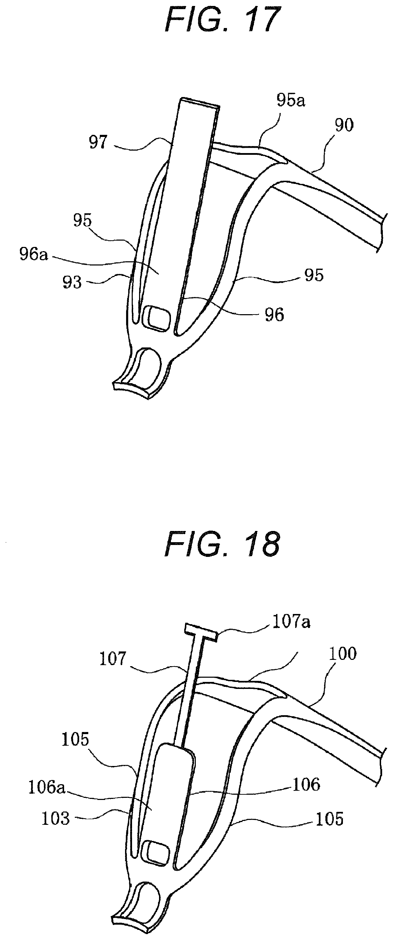

As illustrated in Fig. 17, a distal-side upright portion 93 of an expansion body 90 according to a fourth modification is provided with outer peripheral portions 95 and a back support portion 96. The back support portion 96 has an arm portion 97 extended in the extending direction. The arm portion 97 is spaced apart from outer peripheral portions 95 and other portions of the expansion body 90. When a receiving surface 96a of the back support portion 96 is inclined, the arm portion 97 comes in contact with a merging portion 95a of the outer peripheral portions 95, so that the inclination amount of the back support portion 96 can be limited.

As illustrated in Fig. 18, a distal-side upright portion 103 of an expansion body 100 according to a fifth modification is provided with outer peripheral portions 105 and a back support portion 106. The back support portion 106 has an arm portion 107 extending along the extending direction from a radially outer end. The arm portion 107 is formed into a T shape in which the width of a distal end part 107a increases, and is separated from the outer peripheral portions 105 and the other portion of the expansion body 100. When a receiving surface 106a of the back support portion 106 is inclined, the distal end part 107a of the arm portion 107 comes in contact with a merging portion 105a of the outer peripheral portions 105. Thus, the arm portion 107 can limit the inclination amount of the back support portion 106.

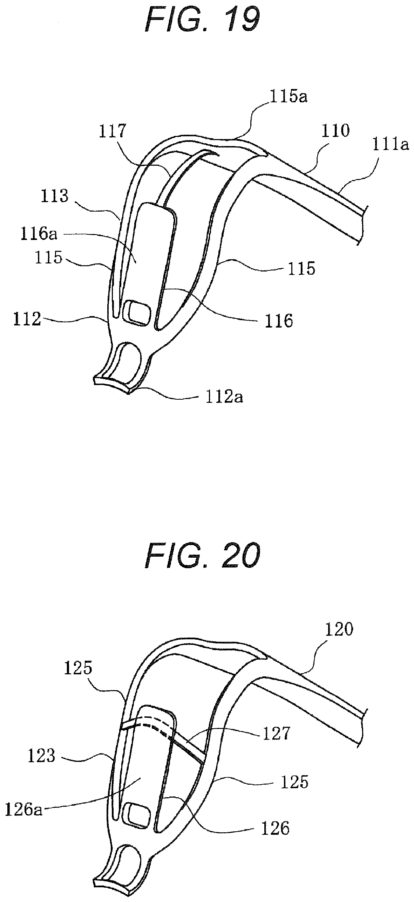

As illustrated in Fig. 19, a distal-side upright portion 113 of an expansion body 110 according to a sixth modification is provided with outer peripheral portions 115 and a back support portion 116. The outer peripheral portions 115 have a merging portion 115a where the outer peripheral portions 115 merge at an end of a recess 112 opposite to a bottom portion 112a. The expansion body 110 has an extension portion 111a extending radially inward from the merging portion 115a of the outer peripheral portions 115. An arm portion 117 extends from the radially outer end of the back support portion 116 toward the inner surface of the extension portion 111a. The distal end of the arm portion 117 is separated from the extension portion 111a. When a receiving surface 116a of the back support portion 116 is inclined, the arm portion 117 comes in contact with the inner surface of the extension portion 111a. Thus, the arm portion 117 can limit the inclination amount of the back support portion 116.

As illustrated in Fig. 20, a distal-side upright portion 123 of an expansion body 120 according to a seventh modification is provided with outer peripheral portions 125 and a back support portion 126. The arm portion 127 extends so as to connect the bifurcated parts of the outer peripheral portions 125. The back support portion 126 is separated from the arm portion 127. When a receiving surface 126a of the back support portion 126 is inclined, a surface of the back support portion 126 opposite to the surface facing the electrode portion 22 comes in contact with the arm portion 127, so that the inclination amount of the back support portion 126 can be limited. As described above, the arm portion 127 may be disposed on the outer peripheral portions 125.

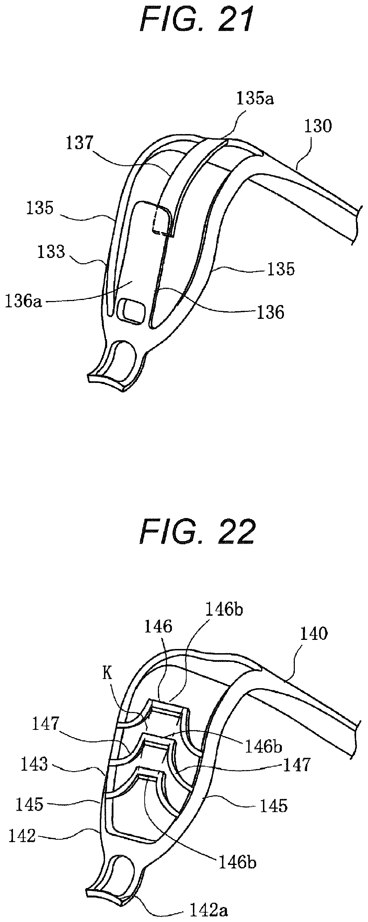

As illustrated in Fig. 21, a distal-side upright portion 133 of an expansion body 130 according to an eighth modification is provided with outer peripheral portions 135 and a back support portion 136. The arm portion 137 extends from a merging portion 135a of the outer peripheral portions 135 toward the back support portion 136. The back support portion 136 is separated from the arm portion 137. When a receiving surface 136a of the back support portion 136 is inclined, a surface of the back support portion 136 opposite to the surface facing the electrode portion 22 comes in contact with the arm portion 137, so that the inclination amount of the back support portion 136 can be limited.

As illustrated in Fig. 22, a distal-side upright portion 143 of an expansion body 140 according to a ninth modification is provided with outer peripheral portions 145. A back support portion 146 includes a plurality of rods 146b extending substantially perpendicular to the radial direction of the expansion body 140 and substantially parallel to the surface of an electrode portion 22 facing a recess 142. The plurality of rods 146b is arranged so as to be substantially parallel to each other. The rods 146b are connected to the outer peripheral portions 145 by connecting arm portions 147 on both sides. The arm portions 147 connect both ends of each of the rods 146b and the outer peripheral portions 145 so as to form a U-shape open to a bottom portion 142a together with the rod 146b. A receiving surface 146a of the back support portion 146 is defined by a virtual plane K defined by the plurality of consecutive rods 146b.

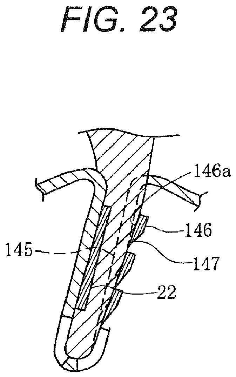

When the receiving surface 146a of the back support portion 146 facing the electrode portion 22 is inclined, the connecting arm portions 147 rotate about the connection portions with the outer peripheral portions 145 as illustrated in Fig. 23. Thus, the inclination amount of the receiving surface 146a is limited.

As described above, the medical device 10 according to the present embodiment includes: the expansion body 21 that is expandable and contractible in the radial direction; the shaft portion 20 that is elongated and includes the distal end part 30 including the proximal end fixing portion 31 to which the proximal end of the expansion body 21 is fixed; and the electrode portion 22 provided along the expansion body 21, in which the expansion body 21 includes the recess 51 that is recessed radially inward during expansion of the expansion body 21 and defines the reception space 51b configured to receive a biological tissue, the recess 51 includes the bottom portion 51a located on an innermost side in the radial direction, the proximal-side upright portion 52 extending outward in the radial direction from the proximal end of the bottom portion 51a, and the distal-side upright portion 53 extending outward in the radial direction from the distal end of the bottom portion 51a, the electrode portion 22 is provided on one of the proximal-side upright portion 52 and the distal-side upright portion 53, another of the proximal-side upright portion 52 and the distal-side upright portion 53 is provided with the outer peripheral portion 55 that is bifurcated from the vicinity of the bottom portion 51a and extends outward in the radial direction, the back support portion 56 that is disposed inside the outer peripheral portion 55 and includes the receiving surface 56a facing the electrode portion 22 when the expansion body 21 expands, and the arm portion 57 extending from the back support portion 56 or from the outer peripheral portion 55, the receiving surface 56a of the back support portion 56 is inclined so as to be substantially parallel to the electrode portion 22 via an object clamped between the electrode portion 22 and the back support portion 56 when the electrode portion 22 moves toward the back support portion 56, and the arm portion 57 includes any one of configurations of i) extending from the back support portion 56, while curving or bending, to be connected to the outer peripheral portion 55, and being deformed to limit an inclination amount of the receiving surface 56a of the back support portion 56 when the receiving surface 56a is inclined, ii) extending from the back support portion 56, and coming into contact with the outer peripheral portion 55 or a part of the expansion body 21 to limit an inclination amount of the receiving surface 56a of the back support portion 56 when the receiving surface 56a is inclined, iii) extending from the outer peripheral portion 55, and coming into contact with the back support portion 56 to limit an inclination amount of the back support portion 56 when the receiving surface 56a of the back support portion 56 is inclined, and iv) connecting the back support portion 56 and the outer peripheral portion 55 so as to form a U-shape open toward the bottom portion 51a together with the back support portion 56, and rotating about a connection portion with the outer peripheral portion 55 to limit an inclination amount of the receiving surface 56a of the back support portion 56 when the receiving surface 56a is inclined. In the medical device 10 configured as described above, the arm portion 57 limits the inclination amount when the receiving surface 56a of the back support portion 56 is inclined. Thus, the back support portion 56 is not excessively inclined, and the pressing force of the electrode portion 22 against the biological tissue when the biological tissue is clamped between the electrode portion 22 and the receiving surface 56a can be adjusted to be appropriate. This makes it possible to cauterize the biological tissue against which the electrode portion 22 is sufficiently pressed, whereby the impedance during conduction of electricity is reliably increased, and it is possible to clearly determine the end of cauterization. In addition, the biological tissue can be effectively cauterized, whereby a shunt having a stable size can be formed. In addition, the electrode portion 22 is in close contact with the biological tissue and the back support portion 56, whereby it is possible to prevent transmission of energy from the electrode portion 22 to blood or other biological tissues.

The back support portion 56 may extend outward in the radial direction in a plate shape from the outer peripheral portion 55 near the bottom portion 51a. With this configuration, the receiving surface 56a of the back support portion 56 can be more reliably inclined substantially parallel to the electrode portion 22.

The arm portion 57 may be bifurcated from the radially outer end of the back support portion 56, and extend symmetrically about an extending direction of the back support portion 56, while curving or bending, to be connected to the outer peripheral portion 55, the arm portion 57 being deformed to limit an inclination amount of the receiving surface 56a of the back support portion 56 when the receiving surface 56a is inclined. This configuration can prevent the receiving surface 56a of the back support portion 56 from being twisted to the left or right when the receiving surface 56a is inclined, whereby the biological tissue can be reliably clamped between the electrode portion 22 and the back support portion 56.

The arm portion 87 may extend from the radially outer end of the back support portion 86, and come into contact with the outer peripheral portion 85 to limit an inclination amount of the receiving surface 86a of the back support portion 86 when the receiving surface 86a is inclined. With this configuration, the arm portion 87 comes in contact with the outer peripheral portion 85, whereby the inclination amount of the receiving surface 86a is reliably limited.

The outer peripheral portion 115 may include the merging portion 115a at which merging occurs at an end opposite to the bottom portion 112a, the expansion body 110 may include the extension portion 111a extending radially inward from the merging portion 115a of the outer peripheral portion 115, and the arm portion 117 may extend from the radially outer end of the back support portion 116, and come into contact with the inner surface of the extension portion 111a to limit an inclination amount of the receiving surface 116a of the back support portion 116 when the receiving surface 116a is inclined. With this configuration, the arm portion 117 comes into contact with the inner surface of the extension portion 111a, whereby the inclination amount of the receiving surface 116a is reliably limited.

The arm portion 127 may extend to connect bifurcated parts of the outer peripheral portion 125, and come into contact with a surface of the back support portion 126 opposite to a surface facing the electrode portion 22 to limit an inclination amount of the receiving surface 126a of the back support portion 126 when the receiving surface 126a is inclined. With this configuration, the inclination amount of the receiving surface 126a is reliably limited by the arm portion 127 provided on the outer peripheral portion 125.

The outer peripheral portion 135 may include the merging portion 135a at which merging occurs at an end opposite to the bottom portion, and the arm portion 137 may extend toward the back support portion 136 from the merging portion 135a of the outer peripheral portion 135, and come into contact with a surface of the back support portion 136 opposite to a surface facing the electrode portion 22 to limit an inclination amount of the receiving surface 136a of the back support portion 136 when the receiving surface 136a is inclined. With this configuration, the inclination amount of the receiving surface 136a is reliably limited by the arm portion 137 extending from the merging portion 135a.

The back support portion 146 may include a plurality of rods 146b extending substantially perpendicular to the radial direction of the expansion body 140 and substantially parallel to a surface of the electrode portion 22 facing the recess 142, the plurality of rods 146b being disposed substantially parallel to each other, the arm portion 147 may include a plurality of connecting arm portions 147 that connect both ends of the plurality of rods 146b and the outer peripheral portion 145 so as to form a U-shape open toward the bottom portion 142a together with the plurality of rods 146b, the receiving surface 146a of the back support portion 146 may be defined by a virtual plane K including at least a part of each of the plurality of rods 146b, and each of the plurality of connecting arm portions 147 may rotate about a connection portion with the outer peripheral portion 145 to limit an inclination amount of the receiving surface 146a of the back support portion 146 when the receiving surface 146a is inclined. With this configuration, the inclination amount of the receiving surface 146a of the back support portion 146 including the plurality of rods 146b is reliably limited by the connecting arm portions 147.

Note that the present invention is not limited to the embodiments described above, and various modifications may be made by those skilled in the art within the technical idea of the present invention.

This application is based on Japanese Application No. 2021-36373 filed on March 8, 2021, the entire content of which is incorporated herein by reference.

### Reference Signs List

- 10: medical device
- 11: guide wire
- 20: shaft portion
- 21: expansion body
- 22: electrode portion
- 23: hand operation unit
- 25: storage sheath
- 26: pulling shaft
- 30: distal end part
- 31: proximal end fixing portion
- 33: distal end fixing portion
- 35: distal member
- 40: housing
- 41: operation dial
- 42: conversion mechanism
- 50: wire portion
- 51: recess
- 51a: bottom portion
- 51b: reception space
- 52: proximal-side upright portion
- 53: distal-side upright portion
- 55: outer peripheral portion
- 56: back support portion
- 56a: receiving surface
- 57: arm portion
- 57a: bent portion

## Claims

1. A medical device comprising:
an expansion body that is expandable and contractible in a radial direction;
a shaft portion that is elongated and includes a distal end part including a proximal end fixing portion to which a proximal end of the expansion body is fixed; and
an electrode portion provided along the expansion body, wherein
the expansion body includes a recess that is recessed radially inward during expansion of the expansion body and defines a reception space configured to receive a biological tissue,
the recess includes a bottom portion located on an innermost side in the radial direction, a proximal-side upright portion extending outward in the radial direction from a proximal end of the bottom portion, and a distal-side upright portion extending outward in the radial direction from a distal end of the bottom portion,
the electrode portion is provided on one of the proximal-side upright portion and the distal-side upright portion,
another of the proximal-side upright portion and the distal-side upright portion is provided with an outer peripheral portion that is bifurcated from a vicinity of the bottom portion and extends outward in the radial direction, a back support portion that is disposed inside the outer peripheral portion and includes a receiving surface facing the electrode portion when the expansion body expands, and an arm portion extending from the back support portion or from the outer peripheral portion,
the receiving surface of the back support portion is inclined so as to be substantially parallel to the electrode portion via an object clamped between the electrode portion and the back support portion when the electrode portion moves toward the back support portion, and
the arm portion includes any one of configurations of
i) extending from the back support portion, while curving or bending, to be connected to the outer peripheral portion, and being deformed to limit an inclination amount of the receiving surface of the back support portion when the receiving surface is inclined,
ii) extending from the back support portion, and coming into contact with the outer peripheral portion or a part of the expansion body to limit an inclination amount of the receiving surface of the back support portion when the receiving surface is inclined,
iii) extending from the outer peripheral portion, and coming into contact with the back support portion to limit an inclination amount of the back support portion when the receiving surface of the back support portion is inclined, and
iv) connecting the back support portion and the outer peripheral portion so as to form a U-shape open toward the bottom portion together with the back support portion, and rotating about a connection portion with the outer peripheral portion to limit an inclination amount of the receiving surface of the back support portion when the receiving surface is inclined.

2. The medical device according to claim 1, wherein the back support portion extends outward in the radial direction in a plate shape from the outer peripheral portion near the bottom portion.

3. The medical device according to claim 2, wherein the arm portion is bifurcated from a radially outer end of the back support portion, and extends symmetrically about an extending direction of the back support portion, while curving or bending, to be connected to the outer peripheral portion, the arm portion being deformed to limit an inclination amount of the receiving surface of the back support portion when the receiving surface is inclined.

4. The medical device according to claim 2, wherein the arm portion extends from a radially outer end of the back support portion, and comes into contact with the outer peripheral portion to limit an inclination amount of the receiving surface of the back support portion when the receiving surface is inclined.

5. The medical device according to claim 2, wherein
the outer peripheral portion includes a merging portion at which merging occurs at an end opposite to the bottom portion,
the expansion body includes an extension portion extending radially inward from the merging portion of the outer peripheral portion, and
the arm portion extends from a radially outer end of the back support portion, and comes into contact with an inner surface of the extension portion to limit an inclination amount of the receiving surface of the back support portion when the receiving surface is inclined.

6. The medical device according to claim 2, wherein the arm portion extends to connect bifurcated parts of the outer peripheral portion, and comes into contact with a surface of the back support portion opposite to a surface facing the electrode portion to limit an inclination amount of the receiving surface of the back support portion when the receiving surface is inclined.

7. The medical device according to claim 2, wherein
the outer peripheral portion includes a merging portion at which merging occurs at an end opposite to the bottom portion, and
the arm portion extends toward the back support portion from the merging portion of the outer peripheral portion, and comes into contact with a surface of the back support portion opposite to a surface facing the electrode portion to limit an inclination amount of the receiving surface of the back support portion when the receiving surface is inclined.

8. The medical device according to claim 1, wherein
the back support portion includes a plurality of rods extending substantially perpendicular to a radial direction of the expansion body and substantially parallel to a surface of the electrode portion facing the recess, the plurality of rods being disposed substantially parallel to each other,
the arm portion includes a plurality of connecting arm portions that connect both ends of the plurality of rods and the outer peripheral portion so as to form a U-shape open toward the bottom portion together with the plurality of rods,
the receiving surface of the back support portion is defined by a virtual plane including at least a part of each of the plurality of rods, and
each of the plurality of connecting arm portions rotates about a connection portion with the outer peripheral portion to limit an inclination amount of the receiving surface of the back support portion when the receiving surface is inclined.
